# EUROPEAN PATENT APPLICATION

(11) **EP 3 461 449 A1**
(43) Date of publication of application: **03.04.2019**
(21) Application number: 17193365.8
(22) Date of filing: 27.09.2017
(51) Int. Cl.: A61B 18/20, A61B 18/00

(54) **CUTTING ELEMENT FOR A HAIR CUTTING DEVICE AND METHOD OF MANUFACTURING THE SAME**

(71) Applicant: Koninklijke Philips N.V., 5656 AE Eindhoven (NL)
(72) Inventor: JURNA, Martin, 5656 AE Eindhoven (NL); VERHAGEN, Rieko, 5656 AE Eindhoven (NL); BOAMFA, Marius Iosif, 5656 AE Eindhoven (NL); THUMMA, Kiran Kumar, 5656 AE Eindhoven (NL); MOESKOPS, Bastiaan Wilhelmus Maria, 5656 AE Eindhoven (NL)
(74) Representative: de Haan, Poul Erik

(57) **Abstract**

According to an aspect, there is provided a cutting element for a hair cutting device, the cutting element comprising a support element; and a first plurality of sections of graded index, GRIN, optical fiber arranged on the support element, wherein the first plurality of sections of GRIN optical fiber are aligned along a first optical axis such that a beam of light entering a first section of GRIN optical fiber propagates through each of the other sections of GRIN optical fiber, and wherein the first plurality of sections of GRIN optical fiber are spaced from each other to form a respective hair cutting region after each section of GRIN optical fiber in which light is focused by the section of GRIN optical fiber.

## Description

### FIELD OF THE INVENTION

The disclosure relates to a cutting element for a hair cutting device and a method of manufacturing the same, and in particular relates to a cutting element that comprises an optical fiber for using light to cut hair.

### BACKGROUND TO THE INVENTION

The use of laser light to cut hair, particularly in a shaving context (for example where a close or clean shave is desired) is seen at present as an attractive alternative to conventional bladed shaving devices. The laser light is used to cut the hair by burning or melting a part of the hair. The use of laser light could provide an improved shaving experience by reducing or eliminating skin irritation (e.g. razor burn) and/or providing a very close shave (e.g. leaving very short, or no, stubble).

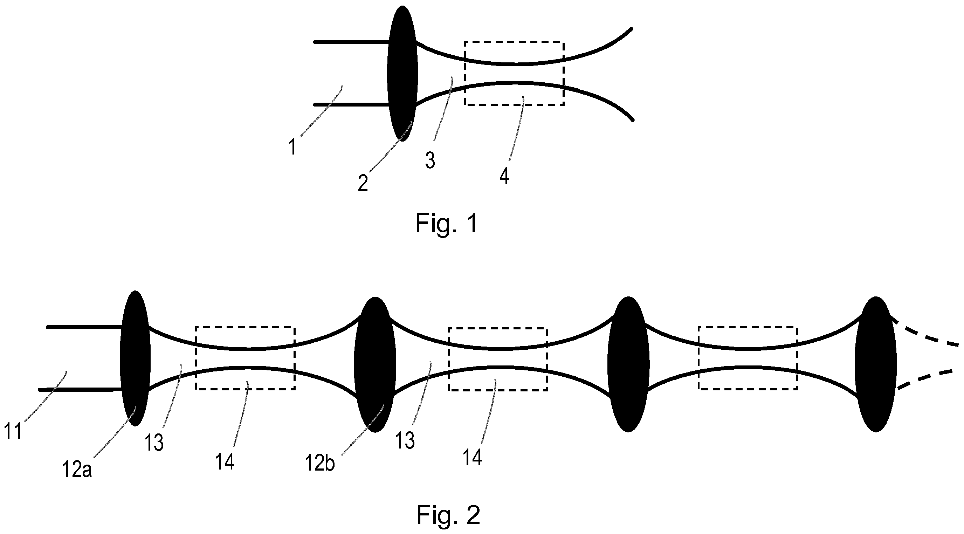

Some devices use a free laser beam approach in which laser light is emitted from an optical fiber or light source and is focused in a free space in the air. An example of the free laser beam approach is shown in Fig. 1. In this Figure, collimated light (e.g. laser light) 1 is incident on a lens 2 which focuses the light (as shown by light 3) to provide focused light in a hair cutting region 4. Hairs within the hair cutting region 4 are heated by the light 3 and 'cut' through the burning of the hair. With this approach, it is relatively easy to provide light at a sufficient intensity to cause the hair to burn, but it is difficult to provide a hair cutting region (i.e. a region in which hairs can be cut) that extends several centimeters (e.g. 4-5 cm as in a conventional bladed razor).

### SUMMARY OF THE INVENTION

One approach to improve the free laser beam approach is to extend the free laser beam geometry with a focused beam that passes through a row of lenses to form a number of hair cutting regions between each of the lenses. This approach is shown in Fig. 2. Thus, collimated light (e.g. laser light) 11 is incident on a lens 12a which focuses the light (as shown by light 13) to provide focused light in a hair cutting region 14. After the hair cutting region 14 the light 13 is incident on a further lens 12b (which is different to lens 12a) that refocuses the light to form another hair cutting region 14, and so on. This approach provides a number of different hair cutting regions 14 and so increases the amount of hair that can be cut. However correctly aligning the lenses 12 in such a system is very difficult and furthermore the tolerances (e.g. x, y, z position and/or spherical curvature of the lenses 12) of every optical element is very high, which increases the complexity and the cost of this type of system.

There is therefore a need to provide further improvements to hair cutting devices and cutting elements that use the free laser beam approach to cut hair.

According to the present disclosure, a plurality of sections of graded index (GRIN) optical fiber are used as the focusing elements in place of conventional lenses. The plurality of sections of GRIN optical fiber can be formed by cutting an optically aligned GRIN optical fiber into separate sections, which means that the sections of GRIN optical fiber are optically aligned with each other. In this way the performance of a cutting element that uses the free laser beam approach can be improved (due to the better alignment of the focusing elements) and the cost and complexity of manufacturing such a cutting element is reduced.

Thus according to a first aspect, there is provided a cutting element for a hair cutting device, the cutting element comprising a support element; and a first plurality of sections of graded index, GRIN, optical fiber arranged on the support element, wherein the first plurality of sections of GRIN optical fiber are aligned along a first optical axis such that a beam of light entering a first section of GRIN optical fiber propagates through each of the other sections of GRIN optical fiber, and wherein the first plurality of sections of GRIN optical fiber are spaced from each other to form a respective hair cutting region after each section of GRIN optical fiber in which light is focused by the section of GRIN optical fiber.

In some embodiments, the support element comprises a plurality of slots, each slot being aligned with a respective space between adjacent pairs of sections of GRIN optical fiber.

In some embodiments, the distance between each section of GRIN optical fiber is between 60 µm and 1 cm, between 60 µm and 1 mm, or between 60 µm and 500 µm.

In some embodiments, the pitch of each section of GRIN optical fiber is between 0.1 and 0.5, or a multiple of a 0.5 step in pitch.

In some embodiments, the cutting element further comprises one or more further pluralities of sections of GRIN optical fiber arranged on the support element, wherein each further plurality of sections of GRIN optical fiber are aligned along a respective further optical axis such that a beam of light entering a first section of GRIN optical fiber in each further plurality propagates through each of the other sections of GRIN optical fiber in the further plurality, and wherein the sections of GRIN optical fiber in each further plurality are spaced from each other to form a respective hair cutting region after each section of GRIN optical fiber in which light is focused by the section of GRIN optical fiber.

In the above embodiments, the hair cutting regions formed by the spaces between the plurality of sections of GRIN optical fiber in a further plurality can be displaced laterally with respect to the hair cutting regions formed by the spaces between the first plurality of sections of GRIN optical fiber, wherein the lateral displacement is relative to a direction of movement of the cutting element during a cutting operation.

According to a second aspect, there is provided a hair cutting device for cutting hair on a subject, the hair cutting device comprising a cutting element as described in any of the above embodiments; and a light source for generating a beam of light, wherein the light source is coupled to an end section of GRIN optical fiber in the cutting element.

According to a third aspect, there is provided a method of manufacturing a cutting element for a hair cutting device, the method comprising forming a support element having a first graded index, GRIN, optical fiber arranged on the support element; cutting the first GRIN optical fiber at a plurality of locations to form a plurality of separated sections of GRIN optical fiber, wherein the space after each section of GRIN optical fiber is a hair cutting region in which a beam of light is focused by the section of GRIN optical fiber.

In some embodiments, the step of forming comprises forming the support element; and attaching the first GRIN optical fiber to the support element.

In some embodiments, the step of forming comprises forming the support element with the first GRIN optical fiber arranged in a straight line along an optical axis such that the plurality of sections of GRIN optical fiber formed by the cutting of the first GRIN optical fiber are aligned along the optical axis.

In some embodiments, the step of cutting comprises cutting the first GRIN optical fiber and the support element at the plurality of locations to form the plurality of separated sections of GRIN optical fiber and a plurality of slots in the support element, each slot being aligned with a respective space between adjacent pairs of sections of GRIN optical fiber.

In some embodiments, the step of cutting the GRIN optical fiber comprises cutting the GRIN optical fiber such that the distance between each section of GRIN optical fiber is between 60 µm and 1 cm, between 60 µm and 1 mm, or between 60 µm and 500 µm.

In some embodiments, the pitch of each section of GRIN optical fiber is between 0.1 and 0.5, or a multiple of a 0.5 step in pitch.

In some embodiments, the method further comprises forming at least one further GRIN optical fiber on the support element; cutting the at least one further GRIN optical fiber at a plurality of locations to form a plurality of separated sections of GRIN optical fiber for each further GRIN optical fiber, wherein the space after each section of GRIN optical fiber is a hair cutting region in which a beam of light is focused by the section of GRIN optical fiber.

In these embodiments, the step of forming the at least one further GRIN optical fiber comprises forming the at least one further GRIN optical fiber with the at least one further GRIN optical fiber arranged in a straight line along a respective optical axis such that the plurality of sections of GRIN optical fiber formed by the cutting of the at least one further GRIN optical fiber are aligned along the respective optical axis.

In some embodiments, the step of cutting comprises cutting the at least one further GRIN optical fiber and the support element at a respective plurality of locations to form the plurality of separated sections of GRIN optical fiber for each further GRIN optical fiber and a respective further plurality of slots in the support element, each slot in the further plurality being aligned with a space between adjacent pairs of sections of the at least one further GRIN optical fiber.

In some embodiments, the step of cutting the at least one further GRIN optical fiber comprises cutting the at least one further GRIN optical fiber at locations that are displaced laterally with respect to the locations at which the first GRIN optical fiber is cut, wherein the lateral displacement is relative to a direction of movement of the cutting element during a cutting operation.

According to a fourth aspect, there is provided a cutting element manufactured according to any of the methods described above.

These and other aspects of the invention will be apparent from and elucidated with reference to the embodiment(s) described hereinafter.

### BRIEF DESCRIPTION OF THE DRAWINGS

Exemplary embodiments of the invention will now be described, by way of example only, with reference to the following drawings, in which:
Fig. 1 illustrates a conventional free laser beam approach to cutting hairs;
Fig. 2 illustrates an improved approach to using a free laser beam to cut hairs;
Fig. 3 is a schematic drawing showing an exemplary hair cutting device;
Fig. 4 is an illustration of the properties of a GRIN optical fiber;
Fig. 5 is an illustration of a cutting element according to an embodiment;
Fig. 6 is a front view of a cutting element according to an embodiment that is being used to cut hair;
Figs. 7(a) and (b) show two exemplary GRIN optical fibers;
Fig. 8 is an illustration of a cutting element according to another embodiment of the invention;
Fig. 9 is a flow chart of a method of manufacturing a cutting element according to an embodiment; and
Figs. 10(a), 10(b) and 10(c) illustrate the manufacture of a cutting element according to an embodiment.

### DETAILED DESCRIPTION OF EMBODIMENTS

As noted above, the invention provides an improvement in the performance of a cutting element that uses the free laser beam approach and improvement in the manufacture of such a cutting element by using a plurality of sections of graded index (GRIN) optical fiber as the focusing elements in place of conventional lenses.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

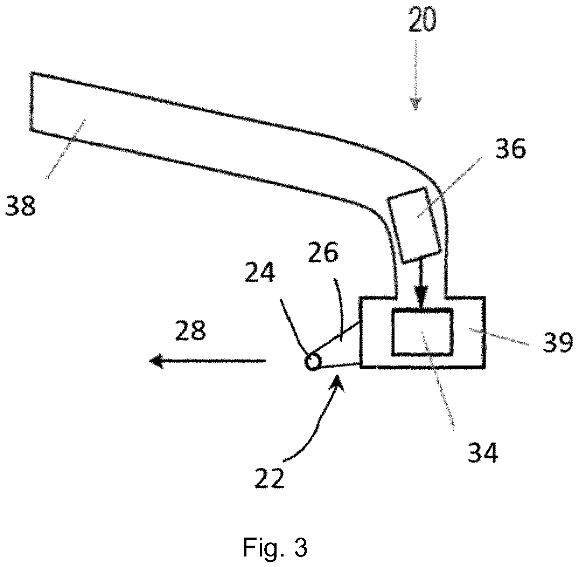

Fig. 3 shows a hair cutting device 20 in the form of a handheld razor according to an exemplary embodiment. The hair cutting device 20 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 20 comprises a cutting element 22 that enables hair to be cut as the hair cutting device 20 is moved over the skin of a subject. The cutting element 22 comprises an optical waveguide 24 and a support element 26 that is arranged on the hair cutting device 20 so that the optical axis of the optical waveguide 24 (i.e. the line along which light typically propagates through the optical waveguide 24) is generally perpendicular to the direction in which the hair cutting device 20 is moved during a hair cutting operation (this direction is indicated by arrow 28).

According to the techniques described herein, the optical waveguide 24 is a plurality of sections of graded index (GRIN) optical fiber that are aligned along a common optical axis. This alignment of the plurality of sections means that a beam of light entering a first section of GRIN optical fiber propagates through each of the other sections of GRIN optical fiber.

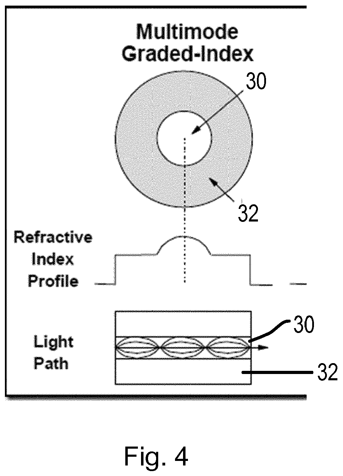

As shown in the top part of Fig. 4, which is a cross-sectional view of a typical GRIN optical fiber, a GRIN optical fiber comprises a core 30 surrounded by cladding 32, which typically fully encompasses the core 30. The middle part of Fig. 4 shows the refractive index profile of the optical fiber, and it can be seen that the core 30 has a refractive index that decreases with increasing radial distance from the optical axis of the GRIN optical fiber (where the optical axis is generally considered to run along the length of the optical fiber through the center of the core 30). Since parts of the core 30 closer to the optical axis have a higher refractive index than the parts near to the cladding 32, light propagating along the optical fiber follows a sinusoidal path through the core 30, as shown in the bottom part of Fig. 4, which is a side cross-sectional view of the optical fiber.

Returning to Fig. 3, a light source 34 is provided in the hair cutting device 20 that generates laser light at one or more specific wavelengths. The light source 34 is optically coupled to a first end of the optical waveguide 24 so that the laser light generated by the light source 34 is coupled into the optical waveguide 24 (and specifically coupled into an end of the optical waveguide 24 so that the laser light propagates through the optical waveguide 24). In some embodiments, the light source 34 can also be coupled to the end of the optical waveguide 24 opposite the first end so that light is input to the optical waveguide 24 at both ends. In embodiments in which the cutting element 22 comprises more than one optical waveguide 24, the light source 34 can be coupled to each optical waveguide 24, or each optical waveguide 24 can have a respective light source 34.

The light source 34 is configured to generate laser light at one or more specific wavelengths that can be used to cut or burn through hair. In particular, each wavelength corresponds to the wavelength of light absorbed by a chromophore that is found in or on hair. As is known, a chromophore is the part of a molecule that provides the molecule with its color. Thus, the laser light will be absorbed by the chromophore and converted into heat which will melt or burn the hair or otherwise destroy the bonds in the molecules of the hair, and it is this melting or burning that provides the cutting action of the hair cutting device 20.

Suitable chromophores that can be targeted by the laser light generated by the light source 34 include, but are not limited to, melanin, keratin and water. Suitable wavelengths of laser light that can be used include, but are not limited to, wavelengths selected from the range 380 nm (nanometers) to 500 nm and 2500 nm to 3500 nm. Those skilled in the art will be aware of the wavelengths of light that are absorbed by these chromophores, and thus also the specific wavelengths of light that the light source 34 should generate for this purpose, and further details are not provided herein.

In some embodiments the light source 34 can be configured to generate laser light at a plurality of wavelengths (either simultaneously or sequentially), with each wavelength being selected to target a different type of chromophore. This can improve the cutting action of the optical waveguide 24 since multiple types of molecules in the hair may be burnt using the laser light. Alternatively multiple light sources 34 can be provided that each generate laser light at a respective wavelength, and each light source 34 can be coupled to a respective optical waveguide 24.

The hair cutting device 20 also comprises a control unit 36 that controls the operation of the hair cutting device 20, and in particular is connected to the light source 34 to control the activation and deactivation of the light source 34, and optionally the power of the light generated by the light source 34. The control unit 36 may activate and deactivate the light source 34 in response to an input from a user of the hair cutting device 20. The control unit 36 can comprise one or more processors, processing units, multi-core processors or modules that are configured or programmed to control the hair cutting device 20. The control unit 36 can also comprise or be associated with a memory or memory module that stores data and computer readable code that is configured to be executed by the control unit 36 to cause the control unit 36 to control the light source 34 and thus enable the hair cutting device 20 to cut hair.

As noted above, Fig. 3 shows a hair cutting device 20 that is in the form of a handheld razor. In addition to the components above, the razor 20 comprises a handle 38 for the subject (or other user of the razor 20) to hold, and a head portion 39 that includes, or connects to, the cutting element 22 (optical waveguide 24 and support element 26). The light source 34 and control unit 36 are shown as being incorporated into the head portion 39 and handle 38 respectively, but it will be appreciated that the positions of these components in the hair cutting device 20 as shown in Fig. 3 is not limiting. Likewise it will be appreciated that the embodiment shown in Fig. 3 is merely an example, and the cutting element 22, light source 34 and control unit 36 can be incorporated or used in place of a conventional blade in any type of hair cutting device 20 that conventionally comprises a blade for physically cutting or slicing hair (whether the blade is static or actuated in order to achieve a cutting action).

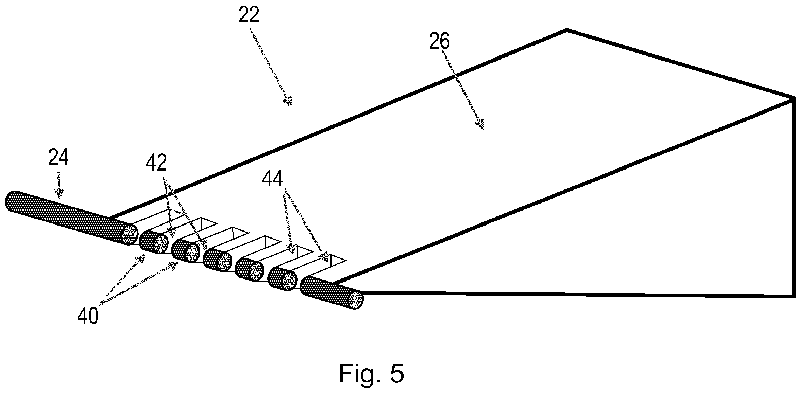

Fig. 5 illustrates a cutting element 22 according to an embodiment of the techniques described herein. As noted above, the cutting element 22 comprises an optical waveguide 24 that comprises a plurality of sections 40 of GRIN optical fiber and a support element 26. Optical fibers are generally fragile, so the support element 26 can provide rigidity and support to the plurality of sections of GRIN optical fiber. The support element 26 can be formed from or made out of any suitable material, including glass, a crystalline or a ceramic material, or a metal. The plurality of sections 40 of GRIN optical fiber can be attached to the support element 26 by any suitable means, including by an adhesive, using laser welding, by providing one or both of the optical waveguide 24 and support element 26 with an attachment structure (e.g. each of the optical waveguide 24 and support element 26 can have part of an interlocking structure), etc.

In some preferred embodiments, a GRIN optical fiber 24 covering the full width of the support element 26 is attached to the support element 26 and the GRIN optical fiber 24 is subsequently cut into the plurality of sections 40. In this way optical alignment of the sections 40 of GRIN optical fiber can be obtained.

In Fig. 5 the support element 26 is shown as having a wedge shape, with the plurality of sections 40 of GRIN optical fiber arranged along the 'thin' end of the wedge shape. However, it will be appreciated that the support element 26 can take any desired shape, and thus the shape of the support element 26 shown in Fig. 5 should not be considered limiting.

Each section 40 in the plurality is spaced from each neighboring section 40. The space 42 after each section 40 of GRIN optical fiber is referred to as a hair cutting region 42. The hair cutting region 40 is the space (i.e. air) into which the light from the preceding section 40 of GRIN optical fiber is focused. It will be appreciated that the reference here to the space 42 *after* each section 40 is in terms of the direction in which light is propagating through the optical waveguide 24. As the cutting element 22 (and hair cutting device 20) is moved across the skin of a subject or user, hairs can enter the hair cutting regions 40 and be heated/burnt to cut the hair.

The plurality of sections 40 of GRIN optical fiber are aligned along a common optical axis such that a beam of light entering a first section 40 of GRIN optical fiber propagates through the first section 40, through the adjacent hair cutting region 42 and into the next section 40, and so on. At least one of the two end sections 40 of GRIN optical fiber can be connected or coupled to the light source 34 to receive light.

In addition to the spaces between the plurality of sections 40 of GRIN optical fiber, slots 44 can be formed in the support element 26. Each slot 44 is aligned with a respective hair cutting region 42 between adjacent pairs of sections 40 of GRIN optical fiber. As described in more detail below, the GRIN optical fiber 24 and support element 26 can be cut during the same operation to form the spaces 42 between the sections 40 and the slots 44 corresponding to the spaces 42. The slots 44 are open to the front of the cutting element 22 (so effectively forming a toothed structure) so that hairs can enter the slots 44 after passing through the hair cutting regions 42. The presence of the slots 44 guides hairs into the slot 44 to be cut.

In the exemplary embodiment of Fig. 5 the optical waveguide 24 comprises seven sections 40 of GRIN optical fiber and a corresponding six hair cutting regions 42. However, it will be appreciated that this number of sections 40 and regions 42 is merely an example, and more or less sections 40 or regions 42 can be provided as required. In addition, as described in more detail below, the number of sections 40/regions 42 may be determined based on the optical and/or physical properties of the GRIN optical fiber, and/or the desired level of focus in the hair cutting regions 42.

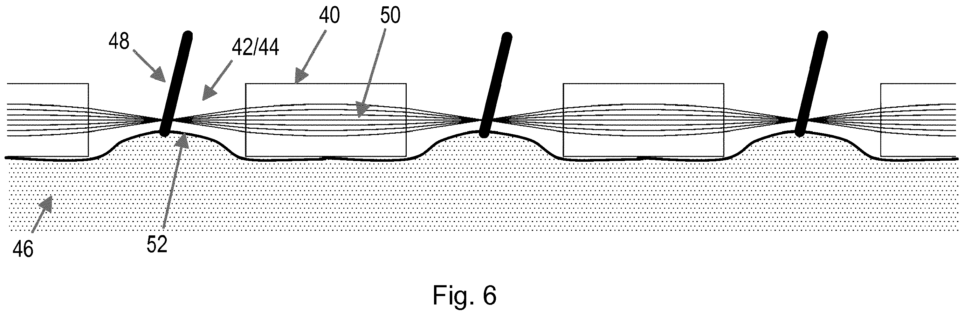

Fig. 6 is a front view of part of the cutting element 22 in Fig. 5 being used to cut hair. Thus, Fig. 6 shows a cutting element 22 placed on the skin 46 of a subject. The skin 46 has a number of hairs 48. As the cutting element 22 is moved across the skin 46, hairs enter the hair cutting regions 42 between the sections 40 of GRIN optical fiber. A light beam 50 is shown propagating through the sections 40 of GRIN optical fiber and the hair cutting regions 42. Due to the optical properties of GRIN optical fibers, the light beam 50 follows a sinusoidal path through the sections 40. Outside of the sections 40 of the GRIN optical fiber, i.e. in hair cutting regions 42, the light beam 50 is focused as a Gaussian beam. Thus, the light beam 50 is focused by each section 40 of GRIN optical fiber such that the light beam 50 is focused in the middle of each hair cutting region 42. The light beam 50 will therefore be focused on any hair 48 in the hair cutting region 42, and the hair can be cut through heating or burning.

It will be appreciated that the arrangement of the sections 40 in Fig. 6 is merely exemplary, and in practice the sections 40 may not achieve the tight light focus shown. However, a tight focus may not be required to cut the hair 48 if the light beam 50 has sufficient power, for example.

It can also be seen in Fig. 6 that placing the cutting element 22 on the skin 46 with some pressure (which can be the typical pressure used with using a conventional bladed cutting device) causes the skin 46 in the areas between the sections 40 of GRIN optical fiber to form domes 52. This doming effect raises the height of any hair 48 on this dome 52 within the hair cutting region 42, enabling the hair to be cut closer to the skin 46, and thereby providing a closer shave. The presence of the slots 44 in the cutting element 22 also helps the domed skin 52 to form. As a rough measure, the height of the domed skin 52 is around one third of the width of the space 42 between the sections 40 of GRIN optical fiber, and this effect can be taken into account when determining the size of the spaces 42 and/or sections 40.

It will be appreciated that the size of the sections 40 of GRIN optical fiber, the optical properties of the sections 40 and the size of the spaces 42 between the sections 40 will determine how focused the light beam 50 is between the sections 40. GRIN optical fibers are commercially available, for example from Thorlabs, Inc. These GRIN optical fibers can have 0.2 and 0.275 Numerical Aperture (NA), and in addition GRIN rod lenses, which can be used as the sections 40 of GRIN optical fiber are commercially available up to 0.55 NA.

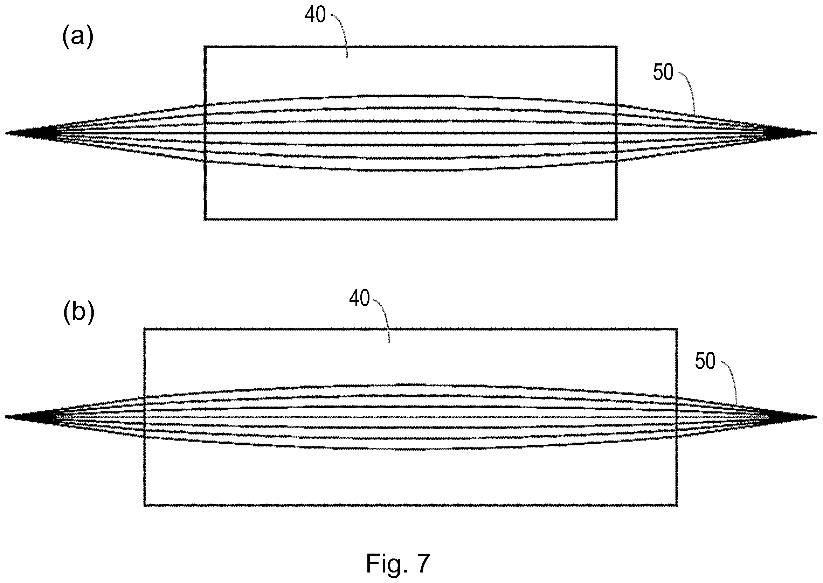

Fig. 7 shows two examples of sections 40 of GRIN optical fibers that can be used in a cutting element 22. The two examples provide for respective slot widths (i.e. respective widths of space 42), where the pitch of the section 40 is different in each example. The pitch of the section 40 is the fraction of a full sinusoidal period that a light beam traverses within the section 40, so for example a section 40 that has a pitch of 0.25 has a length equal to 1/4 of a sine wave period, which would collimate a point light source located at the edge of a section 40). It will be appreciated that different slot widths result in different cutting vs section length ratios. The duty cycle of a plurality of sections 40 of GRIN optical fiber is defined as the ratio between the free beam propagation (i.e. the width of a space 42) and the length of the section 40 of GRIN optical fiber. Thus the duty cycle provides an indication of how much of the width of the cutting element 22 can be used to cut hair. It will be appreciated that the duty cycle is a factor of the beam waist at the focus points of the beam (i.e. in the hair cutting region 42), the pitch and the NA of the sections 40.

In Fig. 7(a), the section 40 of GRIN optical fiber has a pitch of 0.23 and NA of 0.46, which provides a duty cycle of approximately 48%. In Fig. 7(b), the section 40 of GRIN optical fiber has a pitch of 0.29 and NA of 0.46, which provides a duty cycle of approximately 28%.

Thus, based on the above, it can be seen that the size of the sections 40 and/or size of the spaces 42 between the sections 40 can be selected to achieve a desired duty cycle. In some embodiments, the distance between each section 40 of GRIN optical fiber (i.e. the width of each space 42) is between 60 µm and 1 cm (inclusive), but more preferably between 60 µm and 1 mm (inclusive), and even more preferably between 60 µm and 500 µm (inclusive). In some embodiments, the pitch of each section 40 of GRIN optical fiber is between 0.1 and 0.5. In other embodiments, the pitch of each section 40 of GRIN optical fiber is a multiple of a 0.5 step in pitch (e.g. 0.1, 0.6, 1.1, 1.6, ...).

As noted above, the sections 40 may not achieve the tight light focus shown in Fig. 6. In particular, if the requirement for a tight focus is relaxed, the required length of the sections 40 of GRIN optical fiber is reduced, resulting in a higher duty cycle, with the trade-off that the power of the light from the light source 34 will need to increase (unless the cutting rate of the cutting element 22 can be reduced).

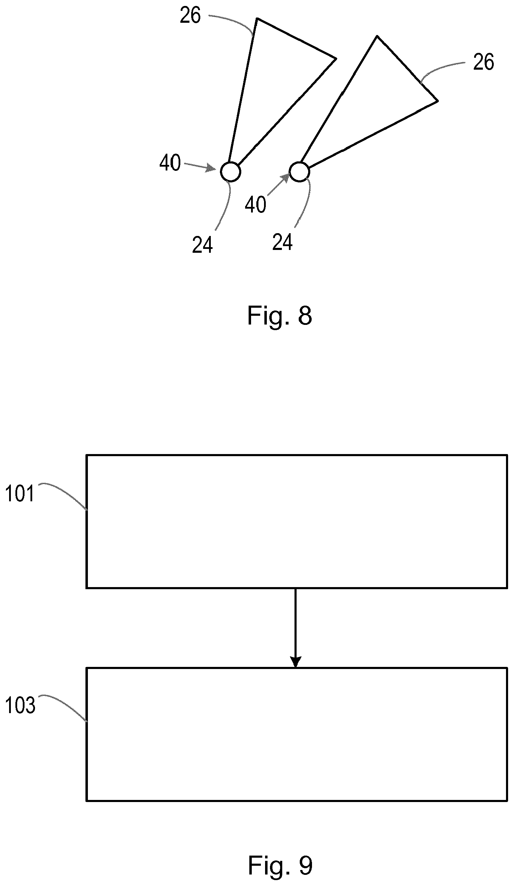

As noted above, the duty cycle provides an indication of how much of the width of the cutting element 22 can be used to cut hair (and so an indication of the probability that the cutting element 22 will catch and cut a particular hair). Since the sections 40 themselves are not used to cut hair, achieving a duty cycle of 100% is not possible with a single optical waveguide 24. Thus, in some embodiments, the duty cycle of the cutting element 22 can be increased by providing multiple pluralities of sections 40 of GRIN optical fiber on the support element 26. In particular, at least a second plurality of sections 40 (i.e. separate from the first plurality of sections 40 shown in Fig. 5) can be arranged ahead or behind the first plurality of sections 40 so that hairs 48 on the skin 46 are first incident on the sections 40 and hair cutting regions 42 of one of the pluralities of sections 40, and then incident on the sections 40 and hair cutting regions 42 of the other plurality of sections 40. Fig. 8 shows a side view of a cutting element having two pluralities of sections 40 that are arranged such that hairs 48 on the skin 46 are first incident on one of the pluralities of sections 40 and then incident on the second one of the pluralities of sections 40. It will be appreciated that although Fig. 8 shows two separate support elements 26, each having one of the pluralities of sections 40 attached, in alternative embodiments the two pluralities of sections 40 can be attached to a single support element 26.

The first plurality of sections 40 and the second plurality of sections 40 have respective optical axes so that a beam of light entering a first section 40 of GRIN optical fiber in the second plurality propagates through each of the other sections 40 of GRIN optical fiber in the second plurality. The respective optical axes may be parallel to each other or substantially parallel to each other.

In some embodiments, the second plurality of sections 40 may have a different pitch and/or slot (space 42) width to the first plurality of sections 40 so as to provide a different duty cycle for that plurality of sections 40 to that provided by the first plurality of sections 40.

In preferred embodiments, the hair cutting regions 42 formed by the spaces between the second plurality of sections 40 are displaced laterally with respect to the hair cutting regions 42 formed by the spaces between the first plurality of sections 40. Displaced laterally is relative to the direction of movement 28 of the cutting element 22 during a hair cutting operation. With this lateral displacement, hairs 48 on the skin 46 that are incident on the sections 40 (rather than incident on the hair cutting regions 42) of a first plurality may be incident on a hair cutting region 42 of the second plurality of sections 40. Thus, with this lateral displacement, hairs that are not cut by the first plurality of sections 40 may be cut by a second or subsequent plurality of sections 40.

In embodiments where the second or subsequent plurality of sections 40 is displaced laterally with respect to another plurality, the sections 40 in each plurality and/or spaces 42 between the sections 40 may have the same or different lengths to the sections 40 and/or spaces 42 in the other pluralities.

It will be appreciated that several (e.g. more than two) pluralities of sections 40 can be provided and arranged so that the hair cutting regions 42 provided by each plurality are displaced laterally with respect to the hair cutting regions 42 provided by the other pluralities to further increase the duty cycle.

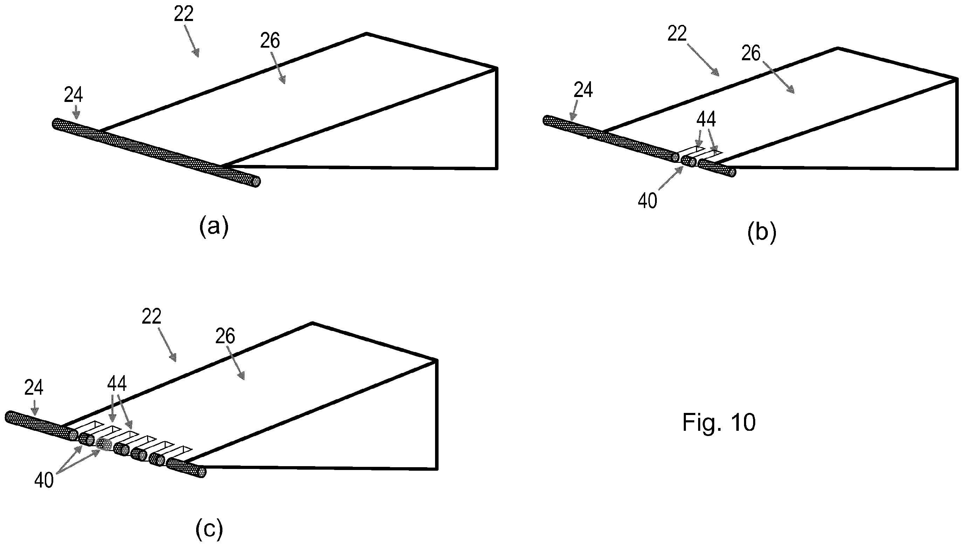

The flow chart in Fig. 9 shows a method of manufacturing a cutting element 22 as described above. Fig. 10 illustrates the manufacturing steps shown in Fig. 9.

In a first step, step 101, a support element 26 having a GRIN optical fiber 24 arranged along the support element 26 is formed. The GRIN optical fiber 24 is preferably arranged in a straight line. Step 101 may comprise forming the support element 26, and then attaching the GRIN optical fiber 24 to the support element 26. In this case, the GRIN optical fiber 24 can be attached to the support element 26 using an adhesive, or any other suitable type of coupling mechanism. The output of step 101 is shown in Fig. 10(a).

Next, in step 103, the GRIN optical fiber 24 is cut at a plurality of locations to form a plurality of separated sections 40 of GRIN optical fiber. This cutting process is shown in Fig. 10(b), with the completed cutting element 22 shown in Fig. 10(c). The plurality of locations at which the GRIN optical fiber 24 is cut form the spaces/hair cutting regions 42. The cutting process in step 103 can comprise a mechanical cutting operation (e.g. cutting using a blade), or it can comprise any other suitable cutting technique.

Preferably, in step 103 the support element 26 is also cut at the plurality of locations to form a plurality of slots 44 in the support element 26, which means that each slot 44 is aligned with a respective space 42 between adjacent sections 40 of GRIN optical fiber. Preferably the cutting of the GRIN optical fiber 24 and the support element 26 is performed in a single process (e.g. using a mechanical cutting operation such as a blade), but alternatively the fiber 24 and support element 26 can be cut using separate processes.

Step 103 can comprise cutting the GRIN optical fiber 24 such that the distance between each section 40 of GRIN optical fiber is between 60 µm and 1 cm (inclusive), between 60 µm and 1 mm (inclusive), or between 60 µm and 500 µm (inclusive). In addition or alternatively, step 103 can comprise cutting the GRIN optical fiber 24 such that the pitch of each section 40 of GRIN optical fiber is between 0.1 and 0.5. In other embodiments, the pitch of each section 40 of GRIN optical fiber is a multiple of a 0.5 step in pitch (e.g. 0.1, 0.6, 1.1, 1.6, ...).

Thus, with this method of manufacture, it is relatively easy to produce a cutting element 22 in which the sections 40 are optically aligned.

In further embodiments of the method, the method can further comprise forming at least one further GRIN optical fiber 24 on the support element 26 and cutting the at least one further GRIN optical fiber 24 at a plurality of locations to form at least one further plurality of separated sections 40 of GRIN optical fiber 24. This step can be performed in order to manufacture a cutting element 22 as shown in Fig. 8.

Preferably, the at least one further GRIN optical fiber is cut at locations that are displaced laterally with respect to the locations at which the first GRIN optical fiber 24 is cut in order to increase the duty cycle of the resulting cutting element 22.

In some embodiments, the support element 26 is also cut to form a further plurality of slots 44 in the support element 26 that are aligned with the space between adjacent sections 40 of the at least one further GRIN optical fiber 24.

Thus, according to embodiments of the techniques described herein, a GRIN optical fiber is processed to create an optically aligned, low cost lens system for free space laser hair cutting.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A cutting element for a hair cutting device, the cutting element comprising:
a support element; and
a first plurality of sections of graded index, GRIN, optical fiber arranged on the support element, wherein the first plurality of sections of GRIN optical fiber are aligned along a first optical axis such that a beam of light entering a first section of GRIN optical fiber propagates through each of the other sections of GRIN optical fiber, and wherein the first plurality of sections of GRIN optical fiber are spaced from each other to form a respective hair cutting region after each section of GRIN optical fiber in which light is focused by the section of GRIN optical fiber.

2. A cutting element as claimed in claim 1, wherein the support element comprises a plurality of slots, each slot being aligned with a respective space between adjacent pairs of sections of GRIN optical fiber.

3. A cutting element as claimed in claim 1 or 2, wherein the distance between each section of GRIN optical fiber is between 60 µm and 1 cm, between 60 µm and 1 mm, or between 60 µm and 500 µm.

4. A cutting element as claimed in any preceding claim, wherein the cutting element further comprises:
one or more further pluralities of sections of GRIN optical fiber arranged on the support element, wherein each further plurality of sections of GRIN optical fiber are aligned along a respective further optical axis such that a beam of light entering a first section of GRIN optical fiber in each further plurality propagates through each of the other sections of GRIN optical fiber in the further plurality, and wherein the sections of GRIN optical fiber in each further plurality are spaced from each other to form a respective hair cutting region after each section of GRIN optical fiber in which light is focused by the section of GRIN optical fiber.

5. A cutting element as claimed in claim 4, wherein the hair cutting regions formed by the spaces between the plurality of sections of GRIN optical fiber in a further plurality are displaced laterally with respect to the hair cutting regions formed by the spaces between the first plurality of sections of GRIN optical fiber, wherein the lateral displacement is relative to a direction of movement of the cutting element during a cutting operation.

6. A hair cutting device for cutting hair on a subject, the hair cutting device comprising:
a cutting element as claimed in any of claims 1-5; and
a light source for generating a beam of light, wherein the light source is coupled to an end section of GRIN optical fiber in the cutting element.

7. A method of manufacturing a cutting element for a hair cutting device, the method comprising:
forming a support element having a first graded index, GRIN, optical fiber arranged on the support element;
cutting the first GRIN optical fiber at a plurality of locations to form a plurality of separated sections of GRIN optical fiber, wherein the space after each section of GRIN optical fiber is a hair cutting region in which a beam of light is focused by the section of GRIN optical fiber.

8. A method as claimed in claim 7, wherein the step of forming comprises:
forming the support element; and
attaching the first GRIN optical fiber to the support element.

9. A method as claimed in claim 7 or 8, wherein the step of forming comprises:
forming the support element with the first GRIN optical fiber arranged in a straight line along an optical axis such that the plurality of sections of GRIN optical fiber formed by the cutting of the first GRIN optical fiber are aligned along the optical axis.

10. A method as claimed in any of claims 7-9, wherein the step of cutting comprises:
cutting the first GRIN optical fiber and the support element at the plurality of locations to form the plurality of separated sections of GRIN optical fiber and a plurality of slots in the support element, each slot being aligned with a respective space between adjacent pairs of sections of GRIN optical fiber.

11. A method as claimed in any of claims 7-10, wherein the method further comprises:
forming at least one further GRIN optical fiber on the support element;
cutting the at least one further GRIN optical fiber at a plurality of locations to form a plurality of separated sections of GRIN optical fiber for each further GRIN optical fiber, wherein the space after each section of GRIN optical fiber is a hair cutting region in which a beam of light is focused by the section of GRIN optical fiber.

12. A method as claimed in claim 11, wherein the step of forming the at least one further GRIN optical fiber comprises:
forming the at least one further GRIN optical fiber with the at least one further GRIN optical fiber arranged in a straight line along a respective optical axis such that the plurality of sections of GRIN optical fiber formed by the cutting of the at least one further GRIN optical fiber are aligned along the respective optical axis.

13. A method as claimed in claim 11 or 12, wherein the step of cutting comprises:
cutting the at least one further GRIN optical fiber and the support element at a respective plurality of locations to form the plurality of separated sections of GRIN optical fiber for each further GRIN optical fiber and a respective further plurality of slots in the support element, each slot in the further plurality being aligned with a space between adjacent pairs of sections of the at least one further GRIN optical fiber.

14. A method as claimed in claim 11, 12 or 13, wherein the step of cutting the at least one further GRIN optical fiber comprises:
cutting the at least one further GRIN optical fiber at locations that are displaced laterally with respect to the locations at which the first GRIN optical fiber is cut, wherein the lateral displacement is relative to a direction of movement of the cutting element during a cutting operation.

15. A cutting element manufactured according to the method of any of claims 7-14.
